# EUROPÄISCHE PATENTSCHRIFT

(11) **EP 1 116 721 B1**
(45) Veröffentlichungstag und Bekanntmachung des Hinweises auf die Patenterteilung: **12.03.2003**
(21) Anmeldenummer: 00100504.0
(22) Anmeldetag: 12.01.2000
(51) Int. Cl.: C07D 475/04

(54) **Verfahren zur Reduktion von Pterinen**
Process for the reduction of pterines
Procédé pour la réduction de ptérines

(43) Veröffentlichungstag der Anmeldung: 18.07.2001
(73) Patentinhaber: Cerbios-Pharma S.A., CH-6917 Barbengo (CH)
(72) Erfinder: Toderi, Nando, 6982 Agno (CH); Marazza, Fabrizio, Dr., 6986 Novaggio (CH)
(74) Vertreter: Zink-Wild, Markus Peter

(56) Entgegenhaltungen:
- EP-A- 0 179 654
- EP-A- 0 608 002
- DE-A- 2 807 393
- TEMPLE C ET AL: "PREPARATION AND PURIFICATION OF L-(PLUS OR MINUS)-5-FORMYL-5,6,7,8-TETRAHYDROFOLIC ACID" JOURNAL OF MEDICINAL CHEMISTRY,US,AMERICAN CHEMICAL SOCIETY. WASHINGTON, Bd. 22, Nr. 6, 1. Juni 1979 (1979-06-01), Seiten 731-734, XP002074295 ISSN: 0022-2623

## Beschreibung

Die vorliegende Erfindung betrifft ein Verfahren zur Reduktion von Pterinen.

In der CH PS 635 344 ist eine Zusammenfassung darüber enthalten, wie man Folsäure zu 5,6,7,8-Tetrahydrofolsäure (nachfolgend abgekürzt mit THF) entweder durch katalytische Hydrierung oder mittels Verwendung von NaBH₄ reduzieren kann.

THF ist das Ausgangsmaterial für die Herstellung von N(5)-Formyl-THF, bekannt als Folinsäure oder Leucovorin, und von N(5)-Methyl-THF.

Diese beiden Verbindungen haben eine grosse pharmakologische Bedeutung als vitaminische Präparate und als Hilfeleistungsmittel (rescue agent) in der Krebs-Chemotherapie; siehe beispielsweise EP 0 409 125.

Sowohl diese katalytische Hydrierung als auch diese Reduktion mittels NaBH₄ ergibt immer stark verunreinigte THF, welche wegen ihrer inhärenten Unstabilität nicht gereinigt werden kann.

Diese herkömmliche Reduktion von Folsäure mit NaBH₄ verlangt einen grossen Ueberschuss an NaBH₄ (5 bis 30 Mol pro Mol Folsäure).

Diese Reduktion läuft nur bei erhöhter Temperatur (60°C bis 80°C) ab.

Wird die so erhaltene, ungereinigte THF zu N(5)-Formyl-THF oder N(5)-Methyl-THF weiterverarbeitet, dann müssen diese Produkte mittels aufwendigen Verfahren gereinigt werden; siehe CH PS 635 344.

C. Temple et al., J. Med. Chem. (1979), 22, 731 beschreibt eine verbesserte NaBH₄-Reduktion von Folsäure.

Diese Reduktion läuft bei Raumtemperatur ab, benötigt aber immer noch einen grossen Ueberschuss an NaBH₄ (etwa 10 Mol pro Mol Folsäure).

Wegen dieses grossen Ueberschusses an NaBH₄ ist es wenig sinnvoll, diese Reduktionsmethode im industriellen Massstab zu verwenden, nicht zuletzt aus Sicherheitsgründen (Explosionsgefahr).

Auch gemäss EP 0 608 002 wird 1 Mol Folsäure mit etwa 10 Mol NaBH₄ reduziert.

Metall-katalysierte NaBH₄-Reduktionen von verschiedenen Substraten, wie Ketone, Ester, Acylhalogenide, aromatische Systeme etc., sind bekannt; siehe "Sodium Borohydride Digest", (1995), 3rd ed., Morton International Inc.

Als Katalysatoren werden beispielsweise Cu(II), Ni(II), Co(II), und Lanthanid-Salze verwendet.

Es ist ein Ziel der vorliegenden Erfindung, ein Verfahren zur Reduktion von Pterinen mit einem Alkalimetailborhydrid zur Verfügung zu stellen, bei welchem das jeweilige Reduktionsprodukt ohne nennenswerte Verunreinigungen erhalten wird.

Bei diesem Verfahren soll nur ein geringer Ueberschuss an Reduktionsmittel verwendet werden.

Dieses Verfahren soll bei tiefer Temperatur, insbesondere im Bereich von 0°C bis 25°C, ablaufen.

Dieses Verfahren soll kostengünsig im industriellen Massstab durchgeführt werden können.

Völlig überraschend ist gefunden worden, dass man die obigen Ziele erreicht, wenn man die Reduktion in Gegenwart einer katalytischen Menge eines wasserlöslichen Pb(II)-Salzes durchführt.

Das erfindungsgemässe Verfahren zur Reduktion von Pterinen der allgemeinen Formel I 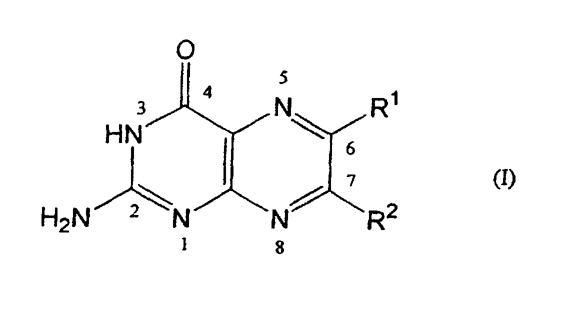 worin
- R¹: Wasserstoff, eine C₁ bis C₃-Alkylgruppe, eine 1,2-Dihydroxypropylgruppe, einen Rest der Formel II 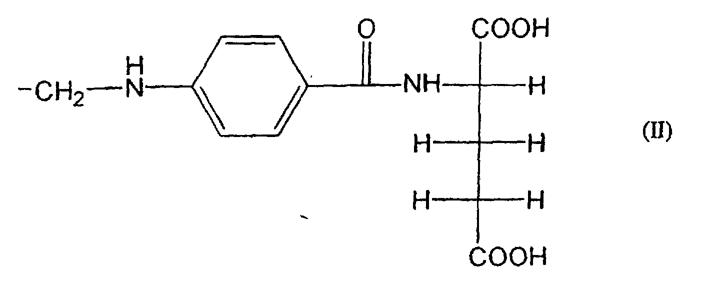
oder
einen Rest der Formel III 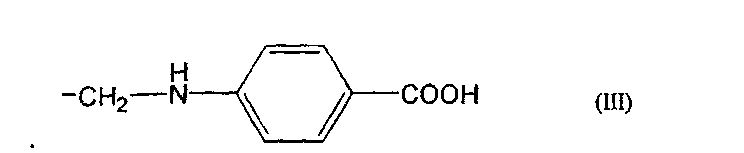 bedeutet, und
- R²: Wasserstoff, eine C₁ bis C₃-Alkylgruppe oder eine 1,2-Dihydroxypropylgruppe bedeutet,
wobei, wenn R² eine 1,2-Dihydroxypropylgruppe ist, R¹ Wasserstoff oder eine C₁ bis C₃-Alkylgruppe sein muss,
mittels einem Alkalimetallborhydrid in Wasser zu 5,6,7,8-Tetrahydropterinen der allgemeinen Formel IV 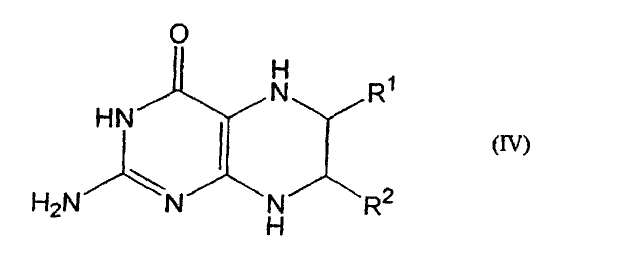 worin
- R¹ und R²: die oben angegebene Bedeutung haben,
ist dadurch gekennzeichnet, dass man die Reduktion in Gegenwart einer katalytischen Menge eines wasserlöslichen Pb(II)-Salzes durchführt.

Bevorzugte Ausführungsformen dieser Erfindung sind in den abhängigen Ansprüchen definiert.

Der im Anspruch 1 enthaltene Proviso dient dazu, unbekannte Verbindungen auszuschliessen.

Der Mechanismus der erfindungsgemässen Pb(II)-katalysierten Reaktion ist bis zum Zeitpunkt der Hinterlegung dieser Erfindung nicht bekannt.

Mit üblichen Uebergangsmetallsalzen, wie beispielsweise Fe(II), Co(II), Ni(II), Cu(II), Zn(II), kann das erfindungsgemässe Verfahren nicht durchgeführt werden.

Die mit dem erfindungsgemässen Verfahren hergestellten Tetrahydropterine haben im allgemeinen eine chromatographische Reinheit von mehr als 95%.

Es ist bevorzugt, mit dem erfindungsgemässen Verfahren aus Folsäure THF herzustellen.

Die so hergestellte THF hatte eine chromatographische Reinheit von mehr als 96 % und eignet sich somit für die Synthese von N(5)-Formyl-THF oder N(5)-Methyl-THF von pharmakologischer Qualität; siehe NO 172 492.

Die nachfolgenden Beispiele sollen die vorliegende Erfindung illustrieren.

### Beispiel 1

16,75 g (41 mmol) Folsäure mit einem Wassergehalt von etwa 8 % wurden in einer Lösung von 17,5 mg (0,053 mmol) Pb(NO₃)₂ in 200 ml deionisierten Wasser suspendiert.

Mittels tropfenweiser Hinzugabe von 20%-iger NaOH unter starker Rührung bei Raumtemperatur wurde der pH-Wert des Gemisches auf 7,5 eingestellt.

Man erhielt eine klare Lösung, welche auf eine Temperatur von 10°C abgekühlt wurde.

Unter Rühren tropfte man bei dieser Temperatur während 20 Minuten eine Lösung von 5,0 g (132 mmol) NaBH₄ in 25 ml deionisiertem Wasser hinzu.

Während dieser Hinzugabe wurde die Temperatur von 10°C beibehalten.

Während dieser Hinzugabe wurde der pH-Wert des Gemisches zwischen 8,5 und 8,8 mittels tropfenweiser Hinzugabe von 20%-iger wässriger Zitronensäure gehalten.

Nach beendeter NaBH₄-Hinzugabe liess man unter Rühren die Temperatur auf 23°C ansteigen und rührte bei dieser Temperatur weitere 2 Stunden.

Anschliessend wurde diese Lösung auf eine Temperatur von 10°C abgekühlt und unter starkem Rühren wurde tropfenweise 18 %-ige HCl so lange hinzugegeben, bis das Gemisch einen pH-Wert von 3,6 aufwies.

Bei diesem Ansäuern fiel die THF aus.

Die ausgefallene THF wurde mittels Filtration isoliert, einmal mit entgastem und deionisiertem Wasser und einmal mit 90 %-igem Ethanol gewaschen.

Der erhaltene Festkörper wurde unter reduziertem Druck getrocknet.

Man erhielt 15,7 g THF.

Eine HPLC-Analyse dieses Produktes zeigte eine Reinheit von 96,3 %.

### Beispiel 2

16,75 g (41 mmol) Folsäure wurden wie in Beispiel 1 beschrieben reduziert, allerdings ohne die Zugabe von Pb(NO₃)₂.

Die HPLC-Analyse des isolierten Produktes zeigte eine Reinheit von 49,0 %.

## Patentansprüche

1. Verfahren zur Reduktion von Pterinen der allgemeinen Formel I 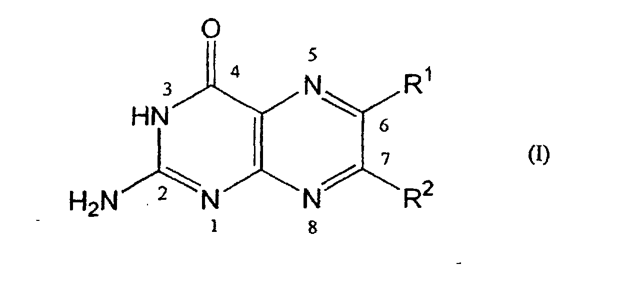 worin
R¹ Wasserstoff, eine C₁ bis C₃-Alkylgruppe, eine 1,2-Dihydroxypropylgruppe, einen Rest der Formel II 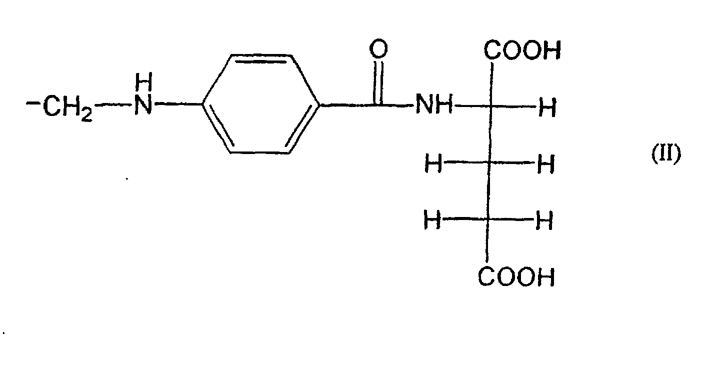 oder einen Rest der Formel III 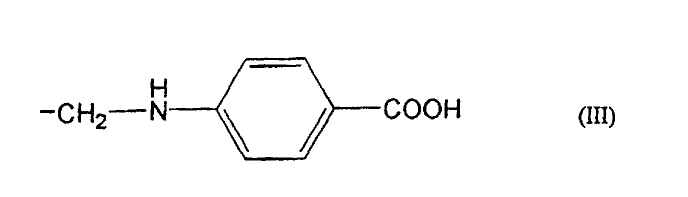 bedeutet, und
R² Wasserstoff, eine C₁ bis C₃-Alkylgruppe oder eine 1,2-Dihydroxypropylgruppe bedeutet,
wobei, wenn R² eine 1,2-Dihydroxypropylgruppe ist, R¹ Wasserstoff oder eine C₁ bis C₃-Alkylgruppe sein muss,
mittels einem Alkalimetallborhydrid in Wasser zu 5,6,7,8-Tetrahydropterinen der allgemeinen Formel IV 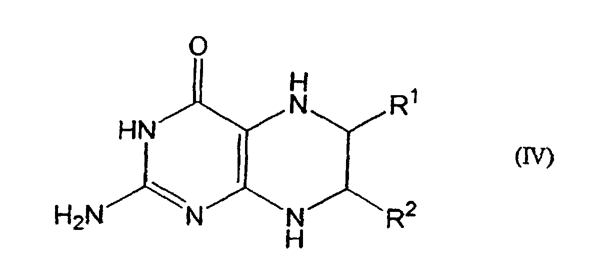 worin
R¹ und R² die oben angegebene Bedeutung haben,
**dadurch gekennzeichnet, dass** man die Reduktion in Gegenwart einer katalytischen Menge eines wasserlöslichen Pb(II)-Salzes durchführt.

2. Verfahren nach Anspruch 1, **dadurch gekennzeichnet, dass** die Reduktion in Gegenwart eines Puffers, beispielsweise Zitronensäure, erfolgt.

3. Verfahren nach einem der Ansprüche 1 bis 2, **dadurch gekennzeichnet, dass** die Reduktion in Gegenwart eines Antioxidationsmittels, beispielsweise Zitronensäure, erfolgt.

4. Verfahren nach einem der Ansprüche 1 bis 3, **dadurch gekennzeichnet, dass** die Reduktion bei einem pH-Wert im Bereich von 7,5 bis 10,0, insbesondere 8,3 bis 8,8, erfolgt.

5. Verfahren nach einem der Ansprüche 1 bis 4, **dadurch gekennzeichnet, dass** die Hinzugabe des Alkalimetallborhydrides, insbesondere Natriumborhydrid, bei einer Temperatur im Bereich von 0°C bis 10°C, insbesondere 5°C bis 10°C, erfolgt, und dass die Vervollständigung der Reduktion bei einer Temperatur von 15°C bis 25°C erfolgt.

6. Verfahren nach einem der Ansprüche 1 bis 5, **dadurch gekennzeichnet, dass** das Molverhältnis von Alkalimetallborhydrid zu reduzierendem Pterin der Formel I von 1,0 bis 3,2, vorzugsweise 2,0, beträgt.

7. Verfahren nach einem der Ansprüche 1 bis 6, **dadurch gekennzeichnet, dass** das Molverhältnis von Pb(II)-Salz zu Alkalimetallborhydrid von 10⁻⁵ bis 10⁻³, vorzugsweise 10⁻⁵, beträgt.

8. Verfahren nach einem der Ansprüche 1 bis 7, **dadurch gekennzeichnet, dass** das Pb(II)-Salz Bleichlorid, Bleiacetat oder Bleinitrat ist.

9. Verfahren nach einem der Ansprüche 1 bis 8, **dadurch gekennzeichnet, dass** die Verbindung der Formel I Folsäure ist.

## Claims

1. A process for the reduction of pterins of the general formula I 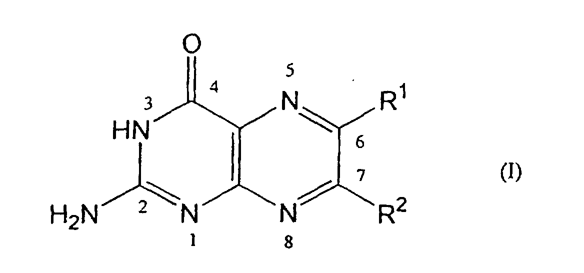 wherein
R¹ is hydrogen, a C₁ to C₃-alkyl group, a 1,2-dihydroxypropyl group, a residue of the formula II 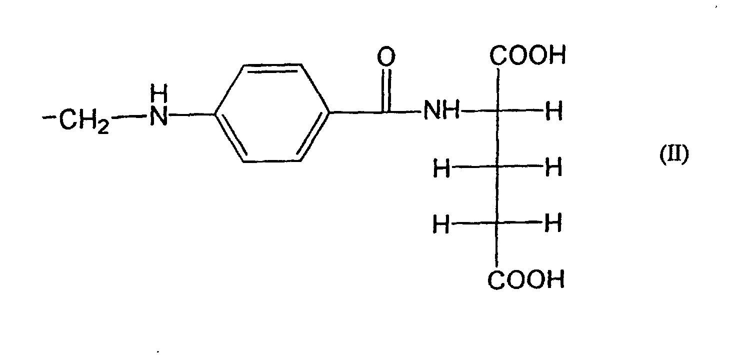
or
a residue of the formula III 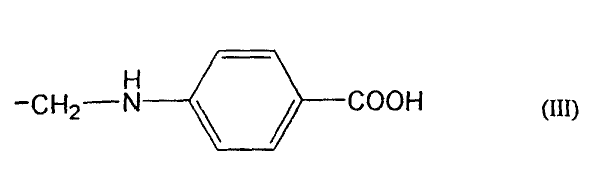 and,
R² is hydrogen, a C₁ to C₃-alkyl group or a 1,2-dihydroxypropyl group,
whereby, when R² is a 1,2-dihydroxypropyl group, R¹ must be hydrogen or a C₁ to C₃-alkyl group,
by means of an alkali metal boron hydride in water to 5,6,7,8-tetrahydropterins of the general formula IV 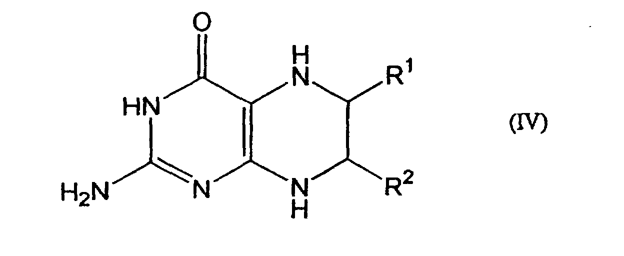 wherein
R¹ and R² have the above given definitions,
**characterized in that** the reduction is carried out in the presence of a catalytical amount of a watersoluble Pb(II)-salt.

2. The process according to claim 1, **characterized in that** the reduction is carried out in the presence of a buffer, for example citric acid.

3. The process according to one of claims 1 to 2, **characterized in that** the reduction is carried out in the presence of an antioxidizing agent, for example citric acid.

4. The process according to one of claims 1 to 3, **characterized in that** the reduction is carried out at a pH-value in the range from 7.5 to 10.0, especially 8.3 to 8.8.

5. The process according to one of claims 1 to 4, **characterized in that** the addition of the alkali metal boron hydride, especially sodium borohydride, is carried out at a temperature in the range from 0°C to 10°C, especially 5°C to 10°C, and that the completion of the reduction is carried out at a temperature from 15°C to 25°C.

6. The process according to one of claims 1 to 5, **characterized in that** the molar ratio from alkali metal boron hydride to the pterin of the formula I to be reduced is from 1.0 to 3.2, preferably 2.0.

7. The process according to one of claims 1 to 6, **characterized in that** the molar ratio from Pb(II)-salt to alkali metal boron hydride is from 10⁻⁵ to 10⁻³, preferably 10⁻⁵.

8. The process according to one of claims 1 to 7, **characterized in that** the Pb(II)-salt is lead chloride, lead acetate or lead nitrate.

9. The process according to one of claims 1 to 8, **characterized in that** the compound of the formula I is folic acid.

## Revendications

1. Procédé pour la réduction de ptérines de la formule générale I : 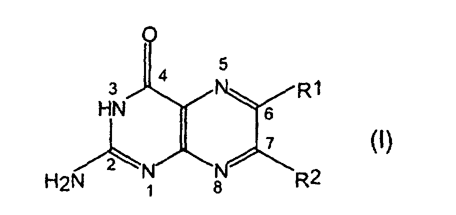 dans laquelle :
R¹ représente l'atome d'hydrogène, un radical alcoyle en C₁ à C₃, le radical 1,2-dihydroxypropyle, un reste de la formule II : 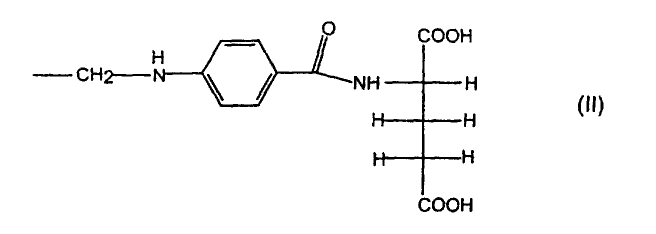
ou un reste de la formule III : 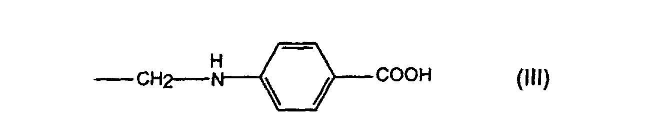 et
R² représente l'atome d'hydrogène, un radical alcoyle en C₁ à C₃, le radical 1,2-dihydroxypropyle,
où lorsque R² est le radical 1,2-dihydroxypropyle, R¹ doit être l'atome d'hydrogène ou un radical alcoyle en C₁ à C₃,
à l'aide d'un borohydrure de métal alcalin dans l'eau en 5,6,7,8-tétrahydroptérines de la formule générale IV : 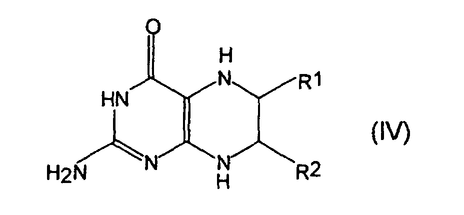 dans laquelle
R¹ et R² ont la signification indiquée ci-dessus,
**caractérisé en ce que** l'on réalise la réduction en présence d'une quantité catalytique d'un sel de Pb(II) soluble dans l'eau.

2. Procédé suivant la revendication 1, **caractérisé en ce que** la réduction est réalisée en présence d'un tampon, par exemple l'acide citrique.

3. Procédé selon l'une quelconque des revendications 1 et 2, **caractérisé en ce que** la réduction est réalisée en présence d'un antioxydant, par exemple l'acide citrique.

4. Procédé selon l'une quelconque des revendications 1 à 3, **caractérisé en ce que** la réduction est réalisée à un pH situé dans l'intervalle allant de 7,5 à 10,0, en particulier de 8,3 à 8,8.

5. Procédé selon l'une quelconque des revendications 1 à 4, **caractérisé en ce que** l'addition du borohydrure de métal alcalin, en particulier du borohydrure de sodium, est réalisée à une température située dans la gamme allant de 0°C à 10°C, en particulier de 5°C à 10°C, et que l'achèvement de la réduction est réalisée à une température allant de 15°C à 25°C.

6. Procédé selon l'une quelconque des revendications 1 à 5, **caractérisé en ce que** le rapport molaire du borohydrure de métal alcalin à la ptérine à réduire de la formule I, se situe dans l'intervalle allant de 1,0 à 3,2, de préférence à 2,0.

7. Procédé selon l'une quelconque des revendications 1 à 6, **caractérisé en ce que** le rapport molaire du sel de Pb(II) au borohydrure de métal alcalin se situe dans l'intervalle allant de 10⁻⁵ à 10⁻³, de préférence à 10⁻⁵.

8. Procédé selon l'une quelconque des revendications 1 à 7, **caractérisé en ce que** le sel de Pb(II) est le chlorure de plomb, l'acétate de plomb ou le nitrate de plomb.

9. Procédé selon l'une quelconque des revendications 1 à 8, **caractérisé en ce que** le composé de formule I est l'acide folique.
